# EUROPÄISCHE PATENTSCHRIFT

(11) **EP 0 546 460 B1**
(45) Veröffentlichungstag und Bekanntmachung des Hinweises auf die Patenterteilung: **12.06.1996**
(21) Anmeldenummer: 92120696.7
(22) Anmeldetag: 04.12.1992
(51) Int. Cl.: A61B 17/58

(54) **Osteosynthesehilfsmittel bei pertrochantärem Oberschenkelhalsbruch**
Osteosynthesis helping device for femoral neck fractures
Dispositif d'aide à l'ostéosynthèse pour fractures du col de fémur

(30) Priorität: 13.12.1991 DE 4141152
(43) Veröffentlichungstag der Anmeldung: 16.06.1993
(73) Patentinhaber: Pennig, Dietmar, Dr. med., D-50935 Köln (DE)
(72) Erfinder: Pennig, Dietmar, Dr. med., D-50935 Köln (DE)
(74) Vertreter: Habbel, Hans-Georg, Dipl.-Ing.

(56) Entgegenhaltungen:
- EP-A- 0 251 583
- EP-A- 0 347 874
- WO-A-91/09571
- US-A- 4 733 654

## Beschreibung

Die Erfindung bezieht sich auf ein Osteosynthesehilfsmittel gemäß dem Oberbegriff des Hauptanspruches und insbesondere bei pertrochantärem Oberschenkelhalsbruch oder bei einem Oberarmbruch.

In der US-PS 47 33 654 wird zur Ruhigstellung des Schenkelhalses und Oberschenkelkopfes ein spezielles Osteosynthesehilfsmittel vorgeschlagen, bei welchem am proximalen Bereich des Marknagels ein zusätzliches Nagelteil festlegbar ist, das eine Bohrung zur Aufnahme einer besonders langen Knochenschraube besitzt. Die Osteosyntheseplatte ist dabei L-förmig ausgebildet und greift mit ihrem kurzen oberen schrägausgerichteten Schenkel in dieses Zusatzteil des Marknagels ein. Dieses Osteosynthesehilfsmittel zwingt also aufgrund der vorgegebenen Bohrungen in dem Marknagel und in dem Zusatzbauteil zu festgelegten Schraubenrichtungen, d. h. die Anbringung und Ausrichtung der Schrauben ist nicht variierbar.

Der Erfindung liegt die Aufgabe zugrunde, eine mechanisch bessere Sicherung der Osteosyntheseplatte dadurch zu schaffen, daß die Richtung der Schrauben frei wählbar ist, so daß damit mit einfachsten Hilfsmitteln eine ausreichende Verankerung und Ruhigstellung des Schenkelhalses erreicht werden kann und der große Rollhügel durch die Osteosyntheseplatte ruhiggestellt wird.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten ausgedrückt wird vorgeschlagen, daß bei einem pertrochantärem Oberschenkelhalsbruch im proximalen Bereich des Oberschenkelknochens ein Marknagel eingesetzt wird, der bis in den großen Rollhügel reicht und an seinem oberen Ende eine - beispielsweise als Schraube am proximalen Ende des Marknagels - ausgebildete Anschlußvorrichtung für eine Osteosyntheseplatte aufweist. Die Osteosyntheseplatte trägt eine Vielzahl von Löchern zur Aufnahme von Knochenschrauben, wobei der transversale Abstand der Löcher größer ist als der Durchmesser des Marknagels, so daß die Schraubenrichtung nicht durch entsprechende Aufnahmebohrungen im Marknagel vorbestimmt wird. Hierdurch können die Löcher so angebracht werden, daß die darin eingesetzten Schrauben beiderseits des Marknagels vorbeiführen und zum Oberschenkelkopf führen, wobei die Richtung der Schrauben frei wählbar ist.

Hierdurch wird eine sichere Festlegung des Schenkelhalses und des Oberschenkelkopfes am proximalen Bereich des Oberschenkelknochens erreicht, wobei dieses sichere Festlegen im wesentlichen mit dem bisher üblichen Instrumentarium erreichbar ist.

Weiterhin wird gemäß der Erfindung vorgeschlagen, daß auch bei einem Oberarmbruch im Bereich des Oberarmkopfes eine Osteosyntheseplatte vorgesehen wird, die an den in den Oberarm eingesetzten Marknagel fest anschließbar ist, wobei weiterhin diese Osteosyntheseplatte mit Bohrungen zur Aufnahme von Schrauben ausgerüstet ist, die so angeordnet sind, daß diese Schrauben am Marknagel vorbeiführen.

Ausführungsbeispiele der Erfindung werden nachfolgend anhand der Zeichnungen erläutert. Die Zeichnungen zeigen dabei in
- Fig. 1: eine Teilansicht auf das proximale Ende eines Oberschenkelknochens, in
- Fig. 2: einen Schnitt gemäß der Linie 2 - 2 in Fig. 1 und in
- Fig. 3: einen Oberarmknochen mit Osteosynthesehilfsmittel.

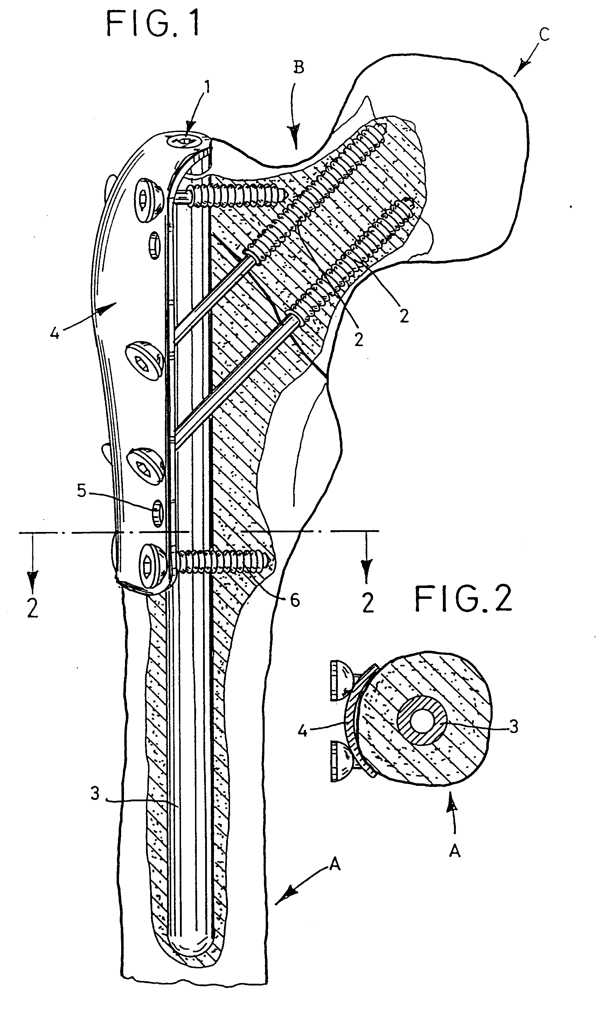

In Fig. 1 ist mit A ein Oberschenkelknochen, mit B der Schenkelhals und mit C der Oberschenkelkopf bezeichnet. In das proximale Ende des Oberschenkelknochens A ist ein Marknagel 3 eingesetzt, der an seinem äußeren oberen Ende einen Gewindeanschluß 1 aufweist, mit dem der Anschluß einer Osteosyntheseplatte 4 an den Marknagel 3 möglich ist. Die Osteosyntheseplatte 4 weist eine Vielzahl von Bohrungen 5 auf, durch die lange Knochenschrauben 2 und kurze Knochenschrauben 6 einsetzbar sind. Die kurzen Knochenschrauben 6 legen dabei die Osteosyntheseplatte 4 an dem Oberschenkelknochen A fest, während die langen Knochenschrauben 2 bis in den Oberschenkelkopf C führen und damit diesen Bereich an dem Oberschenkelknochen festlegen.

Wie dies deutlich die Fig. 1, aber auch deutlich die Fig. 2 zeigt, sind die Bohrungen 5 zur Aufnahme der Schrauben 2 und 6 so angeordnet, daß die eingesetzten Schrauben beiderseits des eingesetzten Marknagels 3 verlaufen und so zum Oberschenkelkopf führen. Hierdurch wird keine direkte Verbindung mit dem Marknagel erforderlich, so daß hier aufwendige Nagelkonstruktionen vermieden werden

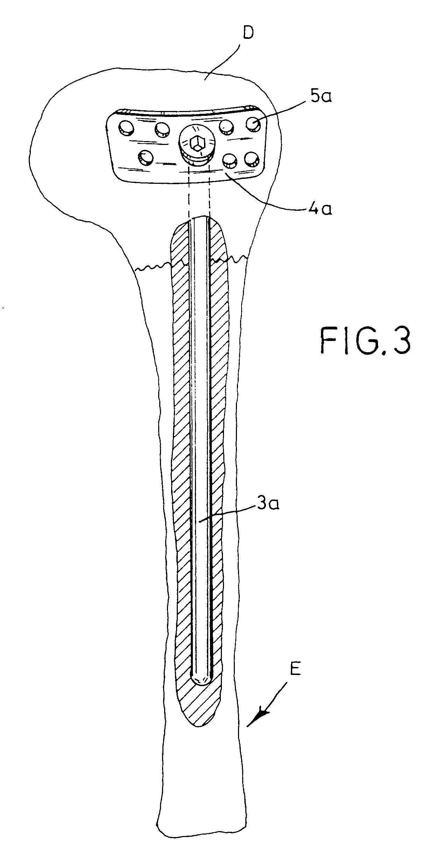

Bei der Ausführungsform gemäß Fig. 3 ist ein Oberarmknochenteil dargestellt mit einem Oberarmkopf D und einem Oberarmknochen E. Am Oberarmkopf D ist eine Osteosyntheseplatte 4a festgelegt, die Bohrungen 5a zur Aufnahme von Festlegschrauben aufweist. Weiterhin kann an diese Osteosyntheseplatte 4a ein Marknagel 3a fest angeschlossen werden, so daß die Lage dieses Marknagels durch die am Oberarmkopf festgelegte Osteosyntheseplatte arretiert wird.

## Patentansprüche

1. Osteosynthesehilfsmittel bei einem Knochenbruch mit einem Marknagel (3) und einer Osteosyntheseplatte (4, 4a), wobei der Marknagel an seinem proximalen Ende einen Anschluß für die Osteosyntheseplatte aufweist und die Osteosyntheseplatte Bohrungen (5, 5a) zur Aufnahme von Schrauben od. dgl. besitzt, dadurch gekennzeichnet, daß die Bohrungen (5, 5a) in der Osteosyntheseplatte (4, 4a) so angeordnet sind, daß die darin eingesetzten Schrauben (2, 6) beiderseits des Marknagels (3) vorbei in den Knochen führen.

2. Osteosynthesehilfsmittel nach Anspruch 1, dadurch gekennzeichnet, daß der bei pertrochantem Oberschenkelhalsbruch zu verwendende Marknagel (3) dazu geeignet ist, vom proximalen Oberschenkelknochen bis in den großen Rollhügel zu reichen und von der Außenseite der Osteosyntheseplatte kurze Knochenschrauben (6), geeignet zur Festlegung der Osteosyntheseplatte (4) in den Oberschenkelknochen führen und daß lange Knochenschrauben (2) von der Außenseite der Osteosyntheseplatte (4) bis in den Schenkelhals (B) führen.

3. Osteosynthesehilfsmittel nach Anspruch 1 oder 2, dadurch gekennzeichnet, daß die Osteosyntheseplatte (4) verformbar und der Knochenkontur anpaßbar ist.

4. Osteosynthesehilfsmittel nach einem der vorhergehenden Ansprüche, dadurch gekennzeichnet, daß die Bohrungen (5) in der Osteosynthesplatte (4) einen transversalen Abstand voneinander aufweisen, der größer ist als der Durchmesser des Marknagels.

5. Osteosynthesehilfsmittel nach Anspruch 1, dadurch gekennzeichnet, daß der bei Oberarmbrüchen zu verwendende Marknagel (3a) eine solche Länge aufweist, daß er vom Oberarmkopf (D) bis in den Oberarmknochen (E) reicht.

## Claims

1. An auxiliary osteosynthesis agent for a bone fracture comprising a medullary nail (3) and an osteosynthetic plate (4, 4a), the medullary nail comprising a connection for the osteosynthetic plate at its proximal end and the osteosynthetic plate comprising holes (5, 5a) for accommodating screws or the like, characterized in that the holes (5, 5a) are so arranged in the osteosynthetic plate (4, 4a) that the screws (2, 6) inserted therein pass into the bone on either side of the medullary nail (3).

2. An auxiliary osteosynthesis agent according to claim 1, characterized in that the medullary nail (3) for use in the case of pertrochanteric femur neck fracture is suited to extending from the proximal femur bone into the greater trochanter, in that short bone screws (6), suited to fixing the osteosynthetic plate (4), extend from the outside of the osteosynthetic plate into the femur bone and in that long bone screws (2) extend from the outside of the osteosynthetic plate (4) into the neck of the femur (B).

3. An auxiliary osteosynthesis agent according to claim 1 or claim 2, characterized in that the osteosynthetic plate (4) is deformable and adaptable to the bone contour.

4. An auxiliary osteosynthesis agent according to any one of the preceding claims, characterized in that the holes (5) in the osteosynthetic plate (4) are transversely spaced from each other, the spacing between them being greater than the diameter of the medullary nail.

5. An auxiliary osteosynthesis agent according to claim 1, characterized in that the medullary nail (3a) for use in the case of upper arm fractures is of such a length that it extends from the head of the upper arm (D) into the humerus (E).

## Revendications

1. Dispositif d'aide à l'ostéosynthèse lors d'une fracture, comprenant un clou centro-médullaire (3) et une plaque d'ostéosynthèse (4, 4a), dans lequel le clou centro-médullaire présente à son extrémité proximale un raccordement pour la plaque d'ostéosynthèse et la plaque d'ostéosynthèse comprend des trous (5, 5a) destinés à la réception de vis ou analogues, caractérisé en ce que les trous (5, 5a) dans la plaque d'ostéosynthèse (4, 4a) sont disposés de telle façon que les vis qui y sont engagées (2, 6) des deux côtés du clou centro-médullaire (3) avancent dans l'os.

2. Dispositif d'aide à l'ostéosynthèse selon la revendication 1, caractérisé en ce que, lors d'une fracture pertrochantérienne du col du fémur, le clou centro-médullaire (3) à utiliser est adapté de manière à aller du fémur proximal jusqu'à la tête fémorale et du côté externe de la plaque d'ostéosynthèse, des vis à os spongieux courtes (6) adaptées à la fixation de la plaque d'ostéosynthèse (4) vont jusque dans le fémur et en ce que des longues vis à os spongieux (2) vont du côté externe de la plaque d'ostéosynthèse (4) jusque dans le col du fémur (B).

3. Dispositif d'aide à l'ostéosynthèse selon la revendication 1 ou 2, caractérisé en ce que la plaque d'ostéosynthèse (4) est modelable et adaptable au contour de l'os.

4. Dispositif d'aide à l'ostéosynthèse selon l'une quelconque des revendications précédentes, caractérisé en ce que les trous (5) de la plaque d'ostéosynthèse (4) sont à une distance transversale les uns des autres qui augmente avec le diamètre du clou centro-médullaire.

5. Dispositif d'aide à l'ostéosynthèse selon la revendication 1, caractérisé en ce que dans les fractures de l'humérus, le clou centro-médullaire (3a) présente une longueur d'utilisation telle qu'il va de la tête de l'humérus (D) jusque dans l'humérus (E).
